# EUROPEAN PATENT SPECIFICATION

(11) **EP 0 323 514 B1**
(45) Date of publication and mention of the grant of the patent: **13.07.1994**
(21) Application number: 88900845.4
(22) Date of filing: 13.01.1988
(51) Int. Cl.: C07C 265/14, C07C 263/00

(54) **PROCESS FOR PREPARING ISOCYANATE COMPOUNDS**
VERFAHREN ZUR HERSTELLUNG VON ISOCYANAT-VERBINDUNGEN
PROCEDE DE PREPARATION DE COMPOSES D'ISOCYANATE

(30) Priority: 13.01.1987 JP 4085/87; 18.06.1987 JP 152032/87; 11.11.1987 JP 284442/87; 16.12.1987 JP 316180/87
(43) Date of publication of application: 12.07.1989
(73) Proprietor: DAICEL CHEMICAL INDUSTRIES, LTD., Sakai-shi Osaka-fu 590 (JP)
(72) Inventor: YAGII, Toyokazu, Hiroshima 739-06 (JP); ITOKAZU,Teruo, Himeji -shi,Hyogo (JP); OKA, Kenji, Hiroshima 739-06 (JP); TANAKA,Yasutaka, Himeji-shi,Hyogo (JP); KOJIMA, Hidetaka, Himeji-shi Hyogo 670 (JP)
(74) Representative: Lightfoot, Robert Oscar
(86) International application number: JP8800026
(87) International publication number: WO8805430

(56) References cited:
- DE-A- 3 504 336
- GB-A- 2 091 730
- JP-A- 5 119 721
- JP-A- 5 488 201
- JP-A-57 158 746
- JP-A-57 158 747

## Description

The present invention relates to a process for the preparation of diisocyanate compounds which comprises a two-stage reaction using an aliphatic diamine and dimethyl carbonate as the starting materials.

An isocyanate compound is an industrially valuable compound, and especially, a bifunctional isocyanate is valuable as the starting material of a polyurethane.

Currently, an isocyanate compound is industrially prepared by reaction between an amine compound and phosgene. As is well-known, phosgene is a reactive compound having a high selectivity, but phosgene has a strong toxicity, and a strict control is necessary when handling this compound. So long as the preparation using phosgene is continued, the risks or damage caused by a leakage of phosgene cannot be avoided.

As the means for preparing an isocyanate compound from an amine compound without using phosgene, there has been proposed a process in which an amine compound is reacted with urea and an alcohol to form a urethane compound and the urethane compound is thermally decomposed in the gas phase (Japanese Unexamined Patent Publication No. 59-205,352 and Japanese Unexamined Patent Publication No. 59-205,353). According to the example of this process, the first step is conducted under a pressure of 6 to 8 bars and the second step is conducted at a reaction temperature of 410°C in the gas phase, and therefore, the equipment cost of this process is high.

A process can be considered in which dimethyl carbonate is used instead of urea and the alcohol. Compared with phosgene, dimethyl carbonate is a safe compound and a special toxicity-removing equipment or inspection is not necessary. Moreover, dimethyl carbonate is prepared from cheap compounds, that is, methanol, carbon monoxide and oxygen (see, for example, Japanese Patent Application No. 61-201,568, Japanese Patent Application No. 61-215,178 and Japanese Patent Application No. 61-215,179).

Accordingly, if an isocyanate compound can be obtained by reacting an amine with dimethyl carbonate to form a urethane compound (first step) and thermally decomposing the urethane compound (second step), an economically advantageous process that can take the place of the phosgene process will be provided.

This reaction is expressed by the following reaction formulae: 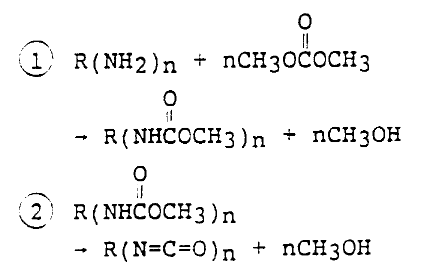
Several processes have been proposed in connection with the first step.

The speed of the reaction between a dialkyl carbonate and an amine compound is very low at a mild temperature under a mild pressure, and a catalyst is necessary to make the process practically utilizable.

The process using a Lewis acid as the catalyst is disclosed in Japanese Examined Patent Publication No. 51-33,095. In the examples of this patent publication, aromatic amines and aliphatic amines are disclosed, but, since the amounts of the catalysts used are large and expensive uranium and antimony are used as the catalysts, the process is not advantageous from the economical viewpoint.

There has been proposed a process in which a urethane compound is prepared by reacting a carbonic acid ester with an amine compound in the presence of a base catalyst (Japanese Unexamined Patent Publication No. 54-163,527). Only examples using aniline which is an aromatic amine are disclosed in this patent publication. In this process, N-methylation as the side reaction is preferential to the urethane-forming reaction, and therefore, the yield of the intended urethane is low.

Another process has been proposed in connection with the reaction to be carried out in the presence of a base catalyst (U.S.P. 4,395,565). Two examples using aniline which is an aromatic amine are disclosed as the example using dimethyl carbonate in this patent publication.

In one of these examples, the reaction is carried out at a temperature (120°C) higher than the boiling point (90°C) of dimethyl carbonate under an increased pressure in an autoclave, but the conversion after 5 hours is only 40% and the methylation product is formed in an amount that cannot be neglected.

In another example, 18.5 g (0.199 mole) of aniline, 22 g (0.244 mole) of dimethyl carbonate, 1.24 g of sodium methylate and 25 ml (17.4 g) of methanol, the total amount being 61.4 g (the concentrations of the components other than methanol are 3.2 moles/kg, 4.0 moles/kg and 2.0% by weight, respectively) are reacted at 70°C under atmospheric pressure for 5 hours. The conversion of aniline is only 15.3% and the space time yield is not satisfactory.

In this patent, it is taught that a temperature range of 100 to 140°C is especially preferred.

In the conventional processes using a base catalyst, dimethyl carbonate is reacted with an amine only in a low yield or space time yield.

Also in connection with the reaction of the second step, several processes have been proposed. For example, Japanese Unexamined Patent Publication No. 59-205,352 and Japanese Unexamined Patent Publication No. 59-205,353 (U.S.P. 4,596,679) propose a process in which decomposition is carried out at a reaction temperature higher than 400°C. But the equipment cost is high because the reaction is preformed in the gas phase, and thus the process is economically disadvantageous.

As the process in which the reaction is carried out in the liquid phase, for example, Japanese Examined Patent Publication No. 57-45,736 (U.S.P. 4,081,472) and Japanese Unexamined Patent Publication No. 51-19,721 (U.S.P. 3,919,279) propose a process in which an isocyanate compound is obtained by thermally decomposing a urethane compound in a high-boiling-point solvent by using a metal or metal salt catalyst. But the catalyst used is a metal or metal salt free of manganese, molybdenum or tungsten and the amount of the catalyst used is relatively large, and since a method in which the catalyst is charged in the high-boiling-point solvent simultaneously with the urethane compound is adopted as the method for adding the catalyst, the reaction efficiency is still low.

Although Japanese Unexamined Patent Publication No. 51-19,721 (U.S. 483,196/A) discloses a process in which thermal decomposition is carried out by using the above-mentioned metal or metal salt as the catalyst, a practical working thereof is economically disadvantageous.

Furthermore, these patent publications disclose only examples directed to the preparation of aromatic isocyanates.

Under this background, development of a process for preparing aliphatic isocyanates having a low reactivity, especially alicyclic isocyanates, economically advantageously is desired.

The present invention seeks to provide a process for the preparation of diisocyanates in which a high pressure or gas phase reaction is not used, the equipment cost is not large, the starting materials used are cheap, and by which the intended product is obtained at a high yield, particularly a high space time yield is good.

The present invention also seeks to provide a high-safety process for the preparation of diisocyanate compounds, which comprises obtaining a urethane compound from an aliphatic diamine and dimethyl carbonate and thermally decomposing the urethane compound.

In accordance with the present invention, there is provided a process for the preparation of an aliphatic diisocyanate compound, which comprises the steps of:
(i) the first-stage reaction of reacting dimethyl carbonate with an aliphatic diamine in the presence of an alkali catalyst to give a corresponding urethane compound, and then
(ii) the second stage reaction of thermally decomposing the urethane compound at a temperature of 150 to 300°C under a reduced pressure of 133.3 to 93,320 Pa (1 to 700 Torr) in a solvent having a boiling point higher than that of the produced diisocyanate compound in the presence of a catalyst composed of at least one metal element selected from manganese, molybdenum, tungsten, zinc and beryllium or a compound of said metal.

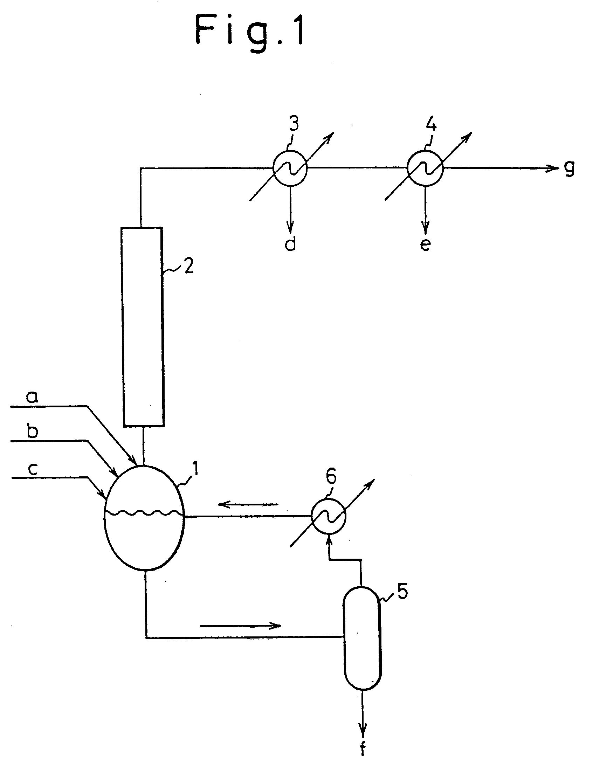

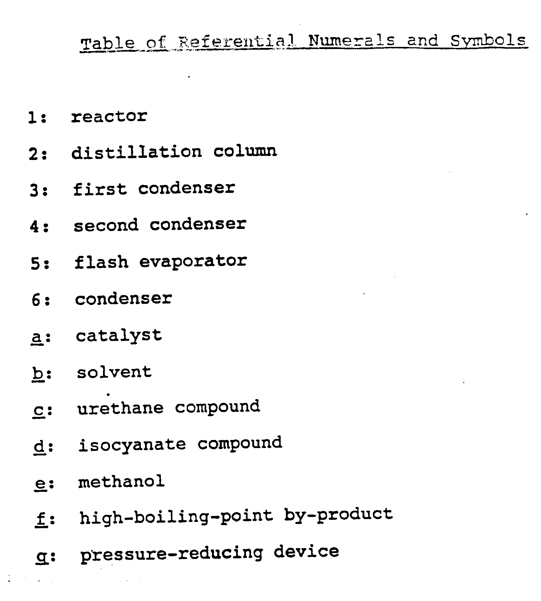

Figure 1 is a block diagram illustrating the flow whereby the second-stage reaction is continuously carried out.

As pointed out hereinbefore, the first-stage reaction of the present reaction advances according to the following reaction formula: 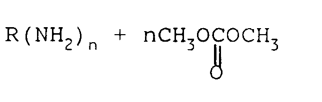
basic substance 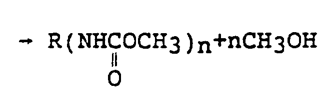
A commercially available product of dimethyl carbonate can be used as it is, or after purification if necessary.

Diamines are classified into aromatic diamines and aliphatic diamines by the chemical reactivity. Aliphatic diamines are used in the present invention. The aliphatic diamines are divided into alicyclic diamines having an alicyclic skeleton in the molecule and linear aliphatic diamines having a chain structure. Linear aliphatic amines are preferred but alicyclic diamines are especially suitable. The following diamines can be mentioned as examples of the diamine that can be used in the present invention.

As the alicyclic diamine, there can be mentioned isophorone diamine, 1,4-diaminocyclohexane, 1,3-bis(aminomethyl)cyclohexane, 1,4-bis(aminomethyl)cyclo-hexane, hydrogenated 4,4-diaminodiphenylmethane and hydrogenated toluylenediamine.

These diamines are valuable because valuable diisocyanates having a cyclic structure can be synthesized therefrom. Especially, the preparation of isophorone diisocyanate, which is a diisocyanate compound having an excellent weatherability, as the intended product from isophorone diamine as the starting material is industrially valuable.

Isophorone diamine includes two isomers, that is, an isomer in which the amino group -NH₂ and the amino-methyl group -CH₂NH₂ are located at the cis-positions in the cyclohexane ring, and another isomer in which the above groups are located at the trans-positions. Each of these isomers can be used as the starting material in the present invention. A mixture of cis- and transisomers, such as commercially available isophorone diamine, can be used without any limitation.

Also, a diamine having amino groups bonded to saturated carbon atoms and an aromatic ring in the skeleton is preferably used as the starting material. As an example of the diamine of this type, there can be mentioned m-xylylenediamine.

As the linear aliphatic diamine, there can be mentioned hexamethylenediamine, 2,2,4-trimethylhexamethylenediamine, 2,4,4-trimethylhexamethylenediamine, tetramethylenediamine and 1,12-diaminododecane.

An aromatic diamine is inferior to an aliphatic diamine in the space time yield or the yield at the urethane formation and therefore, an aromatic diamine is not used in the present invention.

All of the diamines may contain an ether linkage or a stable group such as a sulfone group, a carbonyl group or a halogen group in the skeleton.

Preferably the water content in the diamine to be used is controlled below 1% by weight before the use thereof. This is because water in the diamine, as well as water in the dimethyl carbonate described hereinafter, reduces the activity of the catalyst.

As the basic substance used as the catalyst at the first-stage reaction in the process for preparing diisocyanate compounds according to the present invention, alcoholates of alkali metals and alkaline earth metals are preferred. As the alcoholate, there can be mentioned methylates, ethylates and tertiary butylates of lithium, sodium, potassium, calcium and barium.

Among the basic substances, sodium methylate is especially preferred in view of the availability thereof and for the economical reasons. The amount of sodium methylate used in the present invention is not large enough to cause an economical disadvantage, and a recycling of sodium methylate is not particularly necessary, accordingly, the equipment is simplified. Sodium methylate is commercially available in the form of a methanol solution, which can be easily handled. The basic substance can be used in the form of either a solid or a solution.

A high pressure is not necessary at the first-stage reaction of the preparation process of the present invention, and the reaction is carried out under atmospheric pressure. Nevertheless, the reaction is preferably carried out under such a pressure that will compensate the pressure loss caused by the apparatus structure.

Preferably, the dimethyl carbonate/diamine molar ratio is from 2 to 50 (the dimethyl carbonate/amino group molar ratio is from 1 to 25). More preferably the dimethyl carbonate/diamine molar ratio is at least 4. The reason why the dimethyl carbonate/diamine molar ratio should be controlled to 2 to 50 is that the dimerization reaction is inhibited, and thus, the yield of the urethane compound is improved. If the dimethyl carbonate/diamine molar ratio exceeds 50, the yield of the urethane compound is considerably reduced.

Dimethyl carbonate prepared according to an ordinary process is capable of dissolving a large proportion of water therein, and there is a high risk of a contamination with water. Preferably the water content in dimethyl carbonate used in the present invention is lower than 0.2% by weight. This is because water in dimethyl carbonate reacts with the catalyst to form a metal hydroxide, and the activity of the catalyst is reduced and the amount of the catalyst used must be increased.

The amount of the alkali metal used is determined according to the activity of the catalyst so that the reaction is completed within a reasonably short period of time. For example, sodium methylate is added in an amount such that the content in the crude reaction liquid is 0.001 to 5% by weight, preferably 0.1 to 3% by weight. If the amount of sodium methylate used is smaller than 0.001% by weight, the rate of reaction is low, and if the amount of sodium methylate is larger than 5% by weight, the catalyst is precipitated and the process becomes economically disadvantageous.

The method for carrying out the first-stage reaction is not particularly critical, but in view of a control of the reaction heat, preferably a method is adopted in which the diamine is dropped in dimethyl carbonate.

The reaction temperature can be practically selected in the range of from 0°C to the boiling point of the crude reaction liquid. But, since the rate of reaction is low at a low temperature and the boiling of methanol formed as a by-product is violent at a high temperature, the reaction temperature is preferably within the range of from 30 to 80°C.

A solvent may be used when the starting material is solid or a prevention of precipitation of the formed urethane compound is desired. For example, solvents inactive to the starting material and product, such as methanol, ethanol, tetrahydrofuran, dioxane, benzene and toluene, can be used.

The kind and amount of the solvent are selected so that the starting material or product having a poor solubility is dissolved under the reaction conditions. If the amount of the solvent used is large and the dilution ratio is high, advance of the reaction is retarded, and the process becomes disadvantageous. Preferably the solvent is used in a minimum amount necessary for the dissolution. In general, preferably the solvent is used in an amount of 1 to 10 times the amount of the formed urethane compound.

When the dimethyl carbonate/diamine molar ratio in the starting materials is close to 2, the concentration of the urethane compound formed in the crude reaction liquid is high. Accordingly, if the crystallizability of the urethane compound is high, to eliminate the risk of precipitation, it is necessary to select a solvent having a high dissolving power. If a solvent having a boiling point lower by at least 10°C than the boiling point of the formed urethane compound is used, purification by distillation is facilitated and the process becomes economically advantageous.

Especially in the case of the batchwise reaction, a continuous addition of the catalyst or divided intermittent addition of the catalyst in several times with advance of the first-stage reaction is preferable to collective addition of the catalyst, because the amount of the catalyst used can be reduced to 1/2 to 1/3.

It was found that as shown in the examples given hereinafter, if the catalyst is added continuously or intermittently during the reaction, the necessary amount of the catalyst can be reduced, although the reason therefor is unknown.

If the charging speed of the catalyst for the diamine is high, the boiling of methanol formed as a by-product becomes violent. Accordingly, the charging speed of the catalyst must be controlled, as well as the reaction temperature.

Where the first-stage reaction is carried out in the continuous manner, the catalyst for the diamine can be supplied from not only an inlet in the upstream end portion of a reaction vessel but also an intermediate portion thereof. Furthermore, for example, where the crude reaction liquid is passed through several reactors connected in series, the catalyst can be separately added to the respective reactors.

The formed crude liquid of the urethane compound can be purified to a required purity by an ordinary purification method which includes, for example, consecutive steps of distillation, crystallization, washing with water and then crystallization.

A method in which the alkali component derived from the metal alcoholate as the basic catalyst is neutralized by using an acid such as phosphoric acid and excessive phosphoric acid is then removed by water washing is preferred. If the basic catalyst is heated together with the urethane compound, the urethane compound is further converted to a high-boiling-point compound not intended. Accordingly, this neutralizing step is necessary. The salt formed at the neutralizing step can be removed by an ordinary method such as water washing, filtration or centrifugal separation, and this operation is carried out in combination with the operation of removing methanol. Preferably the formed urethane compound is purified by flash evaporation.

The advantage attained by the use of phosphoric acid at the neutralizing step resides in that, even if the amount of phosphoric acid is too large or too small, the variation of the pH is small and adverse influences are not imposed on the urethane compound at the subsequent purifying step.

Furthermore, preferably a method is adopted in which a crude liquid of the urethane compound is washed with a benzene/water system and the urethane compound is fused and purified by distillation. Benzene is preferred because benzene easily dissolves the urethane compound and the solubility in water is low. The ratio between the amounts used of benzene and water is determined so that the urethane compound is dissolved in benzene at the temperature for the washing treatment of the urethane compound. Generally, benzene is used in an amount of 1 to 10 times the amount of water. Since the solubility differs according to the kind of urethane compound formed, the amount of benzene is appropriately selected within the above-mentioned range according to the kind of urethane compound. Aromatic compounds such as toluene and xylene, and other compounds which are inert and are not soluble in water, such as halogenated hydrocarbons, ether compounds and ester compounds, can be used instead of benzene.

The frequency of washing and the amount of washing liquid are determined so that the amount of water-soluble impurities left in the organic layer is reduced to a predetermined level. The washing can be carried out batchwise or in a continuous manner using an extraction column of the mixer/settler type or the like.

The obtained crude urethane compound can be refined by the flashing method after the neutralization and washing according to the properties of the urethane compound. The degree of this purification has an influence on the yield at the second-stage reaction.

A urethane compound corresponding to the diamine used as the starting material is thus obtained. For example, there can be mentioned 3-methoxycarbonylaminomethyl-3,5,5-trimethyl-1-methoxycarbonylaminocyclohexane, bis(4-methoxycarbonylaminocyclohexyl)methane, 1,4-bis(methoxycarbonylamino)cyclohexane, 1,4-bis-(methoxycarbonylaminomethyl)cyclohexane and 1,3-bis-(methoxycarbonylaminomethyl)cyclohexane.

When these compounds are subjected to thermal decomposition as the second-stage reaction, an elimination of alcohols occurs, and isocyanate compounds corresponding to the skeletons of the starting urethanes, such as isophorone diisocyanate, hydrogenated diphenylmethane-4,4-diisocyanate (MDI), cyclohexane diisocyanate and hydrogenated xylylene diisocyanate (XDI) are formed.

The dimethyl carbonate used for the first-stage reaction of the preparation process of the present invention can be synthesized from phosgene industrially, but the process for directly synthesizing the dimethyl carbonate from carbon monoxide, oxygen and methanol also can be worked. The latter direct synthesis process is more suitable for the process of the present invention, because phosgene is not used.

The thermal decomposition reaction as the second-stage reaction will now be described in detail.

At the second stage of the preparation process of the present invention, the urethane compound obtained by the first-stage reaction is thermally decomposed under a reduced pressure in an inert solvent in the presence of a metal element selected from manganese, molybdenum, tungsten, zinc and beryllium or an inorganic compound or organic compound of said metal, whereby the urethane compound can be converted to a diisocyanate compound in a good yield.

By using the catalyst, a high reaction rate can be attained, and by conducting the reaction under a reduced pressure causing distillation of the formed isocyanate, the isocyanate concentration in the reaction mixture can be maintained at a low level and dimerization or trimerization of the isocyanate group or addition reaction between the isocyanate group and the NH group of the urethane bond, represented by the following formula, can be controlled, and a high yield can be attained: 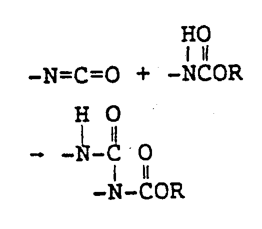
As the compound to be used as the catalyst, there can be mentioned, for example, metallic manganese, manganese oxide (MnO or Mn₂O₃), manganese chloride, manganese sulfate, manganese phosphate, manganese borate, manganese carbonate, manganese acetate, manganese naphthenate, manganese (II) acetylacetonate, manganese (III) acetylacetonate, metallic molybdenum, molybdenum trioxide, molybdenum acetylacetonate [MoO₂(acac)₂], molybdenum dioxide, metallic tungsten, hexacarbonyl tungsten, tungstic anhydride, tungstric acid, zinc bromide and beryllium acetate. These catalysts can be used in the form of a hydrous salt or an anhydride.

Manganese chloride, manganese sulfate, manganese acetate and manganese naphthenate are especially preferred because they are easily commercially available, are cheap, and have a high activity. Manganese acetate is particularly preferred because a required activity is exerted at a low concentration in the crude reaction liquid.

An optimum amount of the catalyst is determined according to the reactivity of the starting material, the temperature and the kind of the catalyst. If the amount of the catalyst is too small, the rate of reaction is low, and if the amount of the catalyst is too large, the amount of a high-boiling-point by-product tends to increase. In general, most preferably the amount of the catalyst in the solvent is 0.0005 to 5% by weight.

The reaction temperature is 150 to 300°C. If the reaction temperature is lower than 150°C, the formation of the isocyanate is retarded and the process is not practically applicable. At a reaction temperature higher than 300°C, working on an industrial scale is difficult and the process becomes industrially disadvantageous.

The solvent must be inert to the isocyanate compound and the urethane compound, and a solvent selected from aliphatic compounds, aromatic compounds, alkylated aromatic compounds and ether compounds can be used. A compound containing an inactive group such as a halogen group can also be used as the solvent.

A solvent that can be purified and separated from the isocyanate compound is preferred. A solvent having a boiling point considerably different from that of the isocyanate is preferred because purification and separation can be performed by distillation. Since a solvent having a boiling point lower than that of the formed isocyanate compound is distilled together with the isocyanate compound and steps are practically complicated, a solvent having a boiling point higher than that of the formed isocyanate is used. A solvent having a boiling point higher by at least 10°C than that of the formed isocyanate compound is especially preferred because the solvent can be easily separated and purified by distillation at the subsequent step.

High-boiling-point by-products are accumulated with the lapse of time, and therefore, a solvent having a boiling point such that regeneration can be industrially performed is preferred.

As preferred examples of the solvent, there can be mentioned o-terphenyl, m-terphenyl, p-terphenyl, a diphenylbenzene mixture, partially hydrogenated triphenyl, dibenzylbenzene, biphenyl, phenyl ether, phenylcyclohexane, hexadecane, tetradecane, octadecane, eicosane, benzyl ether and tetramethylene sulfone.

A suitable solvent must be selected according to the intended isocyanate compound. For example, in the case of the production of isocyanate diisocyanate, partially hydrogenated triphenyl is especially preferred.

The second-stage reaction is carried out under a reduced pressure causing distillation of the formed isocyanate compound from the reaction mixture, whereby the concentration of the isocyanate compound in the reaction mixture is maintained at a low level and a high reaction yield is attained. This effect is especially conspicuous when the reaction is carried out under boiling of the solvent. From this viewpoint, preferably the reaction be carried out under such a reduced pressure that the solvent boils at the adopted reaction temperature. If the degree of reduction of the pressure is too high, recovery of the alcohol formed as the by-product is difficult, and the process becomes disadvantageous in not only the equipment but also the utility. Accordingly, in general, the reaction pressure is at least 133.3 Pa (1 Torr) and lower than 93,320 Pa (700 Torr), preferably lower than 66,666 Pa (500 Torr).

The entire amount of the urethane compound, which is the starting material of the second-stage reaction, may be charged in the solvent at the start of the reaction, but in this case, the amount of the solvent used is large and the process becomes disadvantageous. In view of this point, it is preferable to adopt a continuous reaction method in which the urethane compound is continuously charged in the solvent containing the catalyst, which is boiled under a reduced pressure. In the continuous reaction, preferably the solvent containing 0.0005 to 5% by weight of the catalyst is used in an amount 0.2 to 20 times the amount of the urethane to be treated for every hour based on the weight. If the amount of the solvent exceeds the amount 20 times the amount of the urethane compound, the scale of the apparatus must be undesirably large and the process becomes economically disadvantageous.

Supply of the catalyst in the form of a solution in a solvent such as methanol is preferable to supply of the catalyst in the solid or powdery state, because the amount of the catalyst can be decreased. Manganese acetate is preferred as the catalyst because the solubility in methanol is high.

At the second-stage reaction, the yield of the diisocyanate compound is remarkably improved by using an assistant such as a triester of phosphorous acid as well as the catalyst. As specific examples of the triester of phosphorous acid used as the assistant, there can be mentioned trialkyl esters of phosphorous acid such as triethyl phosphite and tributyl phosphite, and triaryl esters of phosphorous acid such as triphenyl phosphite and tritolyl phosphite. Among these, a high-boiling-point ester such as triphenyl phosphite is advantageous because volatilization during the reaction is small. Since the formation of the isocyanate is inhibited if the amount of the assistant is too large, it is generally preferred that the molar ratio of the assistant to the urethane compound is lower than 1/100, especially lower than 1/1000.

If a reaction apparatus having a structure in which the vapor generated from a reactor is introduced in a distillation column having a reflux device and a fraction rich in the isocyanate compound is obtained in the upper portion of the column is adopted for reducing the amount of the solvent or unreacted starting material incorporated in the formed isocyanate compound, purification of the isocyanate is facilitated and the process becomes advantageous.

Where separation of methanol formed as a by-product from the isocyanate compound is performed, if a first condenser for condensing the isocyanate compound is maintained at a high temperature such that a condensation of methanol does not occur and methanol is condensed in a second condenser maintained at a lower temperature, the isocyanate and methanol can be advantageously separated and recovered. Preferably the first and second condensers are arranged in a vacuum line maintained under a pressure lower than 6,666 Pa (50 Torr). Where methanol is separated and recovered, absorption in an inert solvent may be adopted instead of condensation in the second condenser.

A high-boiling-point by-product is formed in the solvent with the lapse of time, and this by-product causes addition reaction with the isocyanate group of the isocyanate compound as the product, with the result that the yield of the isocyanate compound is reduced.

As means for preventing this disadvantage, there is adopted a method in which the crude liquid mixture in the reactor is continuously withdrawn and subjected to flash evaporation, a high-boiling-point by-product as the residue in the flash evaporator is removed, an evaporated high-boiling-point solvent-rich gaseous mixture is simultaneously condensed, and a liquid mixture of the condensed solvent, the unreacted urethane compound and the isocyanate compound as the product is returned to the reactor.

In this case, the catalyst is lost while being contained in the withdrawn high-boiling-point by-product, and therefore, the catalyst is additionally charged into the reactor in a corresponding amount. The catalyst withdrawn in the state contained in the high-boiling-point by-product can be recovered and used again. But, since the catalyst used in the process of the present invention is relatively cheap and the amount of the catalyst used is small, the running cost is not greatly increased even if the catalyst is discarded.

The layout of the apparatus in which the abovementioned second-stage reaction is carried out in a continuous manner is shown in Fig. 1.

Referring to Fig. 1, a distillation column 2 provided with a reflux device is attached to a reactor 1. A first condenser 3 for condensing the diisocyanate compound and a second condenser 4 for condensing methanol are attached to the distillation column 2. A catalyst a and a solvent b are placed in the reactor 1, and a urethane compound c is charged under a reduced pressure. A fraction rich in an isocyanate compound d is obtained in the upper portion of the distillation column 2, and the isocyanate compound d is condensed from this fraction in the first condenser 3 and a methanol component e is condensed in the second condenser 4. Symbol g represents a pressure-reducing device. The crude liquid mixture in the reactor 1 is continuously withdrawn and subjected to flash evaporation in a flash evaporator 5, and a high-boiling-point by-product f as the residue is removed and the evaporated high-boiling-point solvent-rich gaseous mixture is condensed in a condenser 6 and returned to the reactor 1.

The present invention will now be described in detail with reference to the following examples.

### Referential Example 1

### (Urethane Formation from Aliphatic Amine Compound)

A liquid mixture comprising 74.2 g (0.53 mole) of 3,3,5-trimethylcyclohexylamine, 56.1 g (0.62 mole) of dimethyl carbonate, 4.3 g of 28% sodium methylate and 42.0 g of methanol as the solvent was reacted at 70°C. The concentrations of the respective components were as follows.

| | |
|---|---|
| 3,3,5-trimethylcyclohexylamine: | 3.0 moles/kg |
| dimethyl carbonate: | 3.5 moles/kg |
| sodium methylate: | 0.68% by weight (as pure product) |

After the reaction, the crude reaction liquid was neutralized with acetic acid and analyzed by the gas chromatography. As the result, the presence of 3.7 g of 3,3,5-trimethylcyclohexylamine as the starting material, and 1-methoxycarbonylamino-3,3,5-trimethylcyclohexane was confirmed.

The conversion of the starting amine was 95%, and it is obvious that the process of the present invention is superior to the reaction examples disclosed in the prior art references.

The following examples demonstrate that the yield is high in the process of the present invention.

### Example 1

A round-bottom flask equipped with a stirrer was charged with 85 g of isophorone diamine and 360 g of dimethyl carbonate, and the temperature is elevated to 70°C with stirring in a nitrogen current.

Then, 8.9 g of a 28% solution of sodium methylate in methanol was added dropwise to the mixture over a period of 10 minutes, aging was conducted for 50 minutes and the crude reaction liquid was analyzed by the gas chromatography. It was confirmed that a diurethane compound corresponding to isophorone diamine, that is, isophorone dicarbamate, was formed in a yield of 91% based on isophorone diamine.

### Example 2

The reaction was carried out in the same manner as described in Example 1 except that the reaction temperature was changed to 50°C.

By the analysis of the crude reaction liquid by the gas chromatography, it was confirmed that a diurethane compound corresponding to isophorone diamine, that is, isophorone dicarbamate, was formed in a yield of 77% based on isophorone diamine.

### Example 3

A round-bottom flask equipped with a stirrer was charged with 360 g of dimethyl carbonate, and the temperature was elevated to 70°C with stirring in a nitrogen current.

Then, 8.9 g of a 28% solution of sodium methylate in methanol and 85 g of isophorone diamine were charged into the flask at constant charging rates by using two charging pumps over a period of 2 hours. During this period, the temperature of the crude reaction liquid was maintained at 70°C.

After completion of the charging, aging was conducted at the same temperature for 3 hours, and the crude reaction liquid was neutralized with phosphoric acid and analyzed by the gas chromatography. It was confirmed that a diurethane compound corresponding to isophorone diamine, that is, isophorone dicarbamate, was formed in yields of 99% based on isophorone diamine and 99% based on consumed dimethyl carbonate.

### Example 4

The reaction was carried out in the same manner as described in Example 3 except that 720 g of dimethyl carbonate was used and 16.4 g of a 28% solution of sodium methylate was used.

It was confirmed that the corresponding diurethane compound was quantitatively formed.

### Example 5

The following reactions were carried out by using the same apparatus as used in Examples 1 through 4.

To the reactor maintained at 70°C and charged with 723.1 g of dimethyl carbonate, a mixture of 121.1 g of hexamethylene diamine and 17.3 g of a 28% solution of sodium methylate in methanol was added dropwise dividedly in 4 portions at intervals of 30 minutes.

After the dropwise addition, the mixture was aged at 70°C for 1 hour, titrated with 1/10N HClO₄ , and the amount of HClO₄ consumed by the sodium methylate was corrected whereby it was found that the amine was converted at a conversion of 99.6%.

Then, the crude reaction liquid was neutralized with 7.5 g of 85% phosphoric acid and analyzed by the gas chromatography. It was confirmed that 1,6-bis(methoxycarbonylamino)hexane was formed in a yield of 98.4% based on the diamine.

### Example 6

### (Confirmation of Effect by Divided Charging of Catalyst)

The following reactions were carried out by using the same apparatus as used in Example 1.

### (a) Collective Charging of Catalyst

In the reactor maintained at 70°C and charged with 360 g of dimethyl carbonate, 85 g of isophorone diamine was charged at a constant charging rate by a charging pump over a period of 2 hours. At the start of the reaction, 4.45 g of a 28% solution of sodium methylate as the catalyst in methanol was collectively charged in dimethyl carbonate.

The change of the concentration of isophorone diamine after termination of the charging was traced by measuring the amine value in the crude reaction liquid. The conversion of the amine after correction of the detected amount of sodium methylate was 79.9% after 1 hour and 81% after 2 hours. It was confirmed that the advance of the reaction was slow.

### (b) Divided Charging of Catalyst

The reaction was carried out in the same manner as described above except that 4.45 g of the 28% solution of sodium methylate in methanol was added dropwise dividedly in four portions at intervals of 30 minutes from the start of the charging.

The change of the conversion of the diamine with the lapse of time after completion of the charging of isophorone diisocyanate was such that the conversion after 1 hour was 97.5% and the conversion after 2 hours was 98.6%. It was confirmed that a high effect was obtained by the divided charging of the catalyst.

### Example 7

### (Examination of Catalysts for Second-Stage Reaction)

A compound to be examined with respect to the catalytic activity was charged in a round bottom flask together with 30 g of isophorone dicarbamate, and the flask was immersed in an oil bath maintained at 210°C under a reduced pressure of 4,000 Pa (30 Torr) to effect heating.

After immersion for one hour, the content in the flask was analyzed by using a gas chromatograph.

Kinds and amounts of compounds admitted to have an activity and the analysis results are shown in Table 1.

**Table 1**

| Compound | Amount Added (g) | IPDI (% by weight) | IPMI (% by weight) | IPDC (% by weight) |
|---|---|---|---|---|
| Blank (Comparative) | - | 1.2 | 2.6 | 96.2 |
| MoS₂ | 0.15 | 1.1 | 20.6 | 74.3 |
| MoO₂·(acac)₂ | 0.15 | 4.9 | 36.6 | 45.6 |
| MoO₃ | 0.15 | 1.4 | 6.6 | 92.0 |
| Metallic Mo | 0.3 | 1.0 | 16.9 | 74.4 |
| WO₃ | 0.3 | 1.5 | 9.1 | 88.0 |
| Mn(CH₃CO₂)₂·4H₂O | 0.3 | 9.0 | 32.9 | 39.2 |
| Mn(CH₃CO₂)₂ | 0.3 | 26.9 | 30.2 | 11.8 |
| Mn(H₂PO₄)·4H₂O | 0.3 | 7.2 | 32.9 | 43.1 |
| Mn(acac)₂ | 0.3 | 22.3 | 17.9 | 4.7 |
| Mn(acac)₃ | 0.3 | 15.1 | 32.6 | 23.2 |
| MnH₄(BO₃)₂ | 0.3 | 1.5 | 17.0 | 74.2 |
| MnCO₃ | 0.3 | 12.4 | 42.2 | 32.2 |
| MnCl₂·4H₂O | 0.3 | 22.8 | 37.5 | 20.1 |
| Mn naphthenate (containing 10% by weight of Mn) | 3.0 | 13.5 | 8.1 | 2.5 |
| MnSO₄ hydrate | 0.3 | 31.7 | 40.6 | 17.3 |
| Metallic Mn | 0.3 | 1.4 | 14.5 | 77.0 |
| Abbreviations IPDI: 3-isocyanatomethyl-3,3,5-trimethylcyclohexyl diisocyanate (isophorone diisocyanate) IPMI: 3-isocyanatomethyl-3,5,5-trimethyl-1-methoxycarbonylaminocyclohexane or 3-methoxycarbonylaminomethyl-3,3,5-trimethylcyclohexyl isocyanate (isophorone monoisocyanate) IPDC: 3-methoxycarbonylaminomethyl-3,3,5-trimethyl-1-methoxycarbonylaminocyclohexane (isophorone dicarbamate) | | | | |

In Table 1, isophorone monoisocyanate (IPMI) is a precursor of intended isophorone diisocyanate (IPDI), and this precursor is converted to IPDI by further advance of the reaction.

Accordingly, the effect of the catalyst for thermal decomposition is not evaluated based on the concentration of IPDI but based on the amount contained of unreacted IPDC. Namely, a smaller amount of unreacted IPDC indicates a higher effect.

### Example 8

A 300-ml flask was set at a 10-stage glass Oldar-Show column equipped with a reflux device, and 100 g of Therm S900 Solvent (partially hydrogenated triphenyl) supplied by Nippon Steel Chem. Co. and 0.05 g of manganese acetate tetrahydrate were charged and heated under a reduced pressure. The degree of reduction of the pressure was adjusted so that the liquid boiled at 230°C.

Then, a urethane compound was dropped into the flask at a rate of 60 g/hr from a double-tube type dropping funnel heated at a urethane-compound-flowing temperature by a heating medium. After initiation of the charging, a liquid rich in isophorone diisocyanate rose in the column, and this liquid was distilled from the column top at a distillation rate corresponding to the rate of dropping the urethane.

After, 2 hours' operation, distillation was continued until generation of the isocyanate ceased, and then the operation was stopped. The distillate was collected and the yield was determined from the concentration of isophorone diisocyanate by the gas chromatography. It was confirmed that the yield was 93% based on the starting urethane.

### Examples 9 through 14

The reaction was carried out in the same manner as described in Example 8 except that the kinds of the catalyst and solvent were changed.

After completion of the reaction, the solvent in the flask was analyzed by a gas chromatograph to determine the residual amount of unreacted IPDC.

The results are shown in Table 2.

**Table 2**

| Example No. | Solvent | Catalyst | Amount Added (g) | IPDI | IPMI | IPDC |
|---|---|---|---|---|---|---|
| | | | (mole% based on charged IPDC) | | | |
| 9 | Therm S900 | Mn(CH₃CO₂)₂·4H₂O | 0.05 | 93 | 1 | 0 |
| 10 | Therm S900 | MnCl₂·4H₂O | 0.5 | 67 | 2 | 0 |
| 11 | Therm S900 | MnCO₃ | 0.5 | 26 | 41 | 22 |
| 12 | Therm S900 | MoO₂(acac)₂ | 0.5 | 64 | 5 | 23 |
| 13 | Therm S900 | MnSO₄ hydrate | 0.5 | 46 | 32 | 15 |
| 14 | m-terphenyl | ZnBr₂ | 0.5 | 81 | 13 | 0 |
| Abbreviations IPDI: 3-isocyanatomethyl-3,3,5-trimethylcyclohexyl diisocyanate (isophorone diisocyanate) IPMI: 3-isocyanatomethyl-3,3,5-trimethyl-1-methoxycarbonylaminocyclohexane or 3-methoxycarbonylaminomethyl-3,5,5-trimethylcyclohexylisocyanate (isophorone monoisocyanate) IPDC: 3-methoxycarbonylaminomethyl-3,3,5-trimethyl-1-methoxycarbonylaminocyclohexane (isophorone dicarbamate) | | | | | | |

### Example 15

The reaction was carried out in the same manner as described in Example 8 except that 200 g of Therm S900 was charged.

The presence of IPDI and IPMI in yields of 97% and 3%, respectively, based on charged IPDC in the distillate was confirmed.

### Example 16

The reaction was carried out in the same manner as described in Example 8 except that 120 g of 1,6-bis-(methoxycarbonylamino)-hexane was charged instead of IPDC.

The presence of hexamethylene diisocyanate and the corresponding monoisocyanate in yields of 55% and 19%, respectively, based on charged 1,6-bis(methoxycarbonylamino)hexane in the distillate was confirmed.

### Example 17

The reaction was carried out in the same manner as described in Example 8 except that 200 g of Therm S900 was charged and 17.9 g of a methanol solution containing 0.05% by weight of anhydrous manganese acetate was used as the catalyst instead of 0.05 g of manganese acetate tetrahydrate.

The presence of IPDI and IPMI in yields of 92% and 6%, respectively, based on charged IPDC in the distillate was confirmed.

### Example 18

A glass reaction vessel equipped with a 20-staged Oldar-Show distillation column was charged with 50.0 g of isophorone methyl dicarbamate, 0.1 g of beryllium acetate as the catalyst and 0.1 g of triphenyl phosphite was the assistant. Reaction was carried out at a temperature of 220 to 230°C under a pressure of 666 Pa (5 Torr) for 2 hours while separately distilling isophorone diisocyanate and methanol. By the gas-chromatographical analysis of the distillate and residual liquid, it was confirmed that 38.0 g of isophorone diisocyanate was formed (the yield was 98%).

For comparison, thermal decomposition of isophorone methyl dicarbamate was carried out in the same manner as described above except that triphenyl phosphite was not used. By the gas-chromatography analysis of the distillate and residual liquid, it was confirmed 34.9 g of isophorone diisocyanate was formed (the yield was 90%).

### INDUSTRIAL APPLICABILITY

According to the present invention, a diisocyanate can be obtained from a diamine and dimethyl carbonate in a good yield without using virulently poisonous phosgene. This diisocyanate is valuable as the starting material for the production of polyurethanes, medicines, agricultural chemicals and the like.

## Claims

1. A process for the preparation of an aliphatic diisocyanate compound, which comprises the steps of:
(i) reacting dimethyl carbonate with an aliphatic diamine in the presence of an alkali catalyst to give a corresponding urethane compound, and then
(ii) thermally decomposing the urethane compound at a temperature of 150 to 300ºC under a reduced pressure of 133.3 to 93,320 Pa (1 to 700 Torr) in a solvent having a boiling point higher than that of the produced diisocyanate compound in the presence of a catalyst composed of at least one metal element selected from manganese, molybdenum, tungsten, zinc and beryllium or a compound of said metal.

2. A process according to claim 1, wherein the dimethyl carbonate is prepared by reacting carbon monoxide and oxygen with methanol.

3. A process according to claim 1 or claim 2, wherein the aliphatic diamine is a cyclic diamine.

4. A process according to any of claims 1 to 3, wherein the aliphatic diamine is isophorone diamine.

5. A process according to any of claims 1 to 4, wherein the alkali catalyst used in step (i) is sodium methylate.

6. A process according to claim 5, wherein sodium methylate used as the catalyst is continuously added or intermittently added.

7. A process according to any of claims 1 to 6, wherein the charged dimethyl carbonate/aliphatic diamine molar ratio used in step (i) is from 2 to 50.

8. A process according to any of claims 1 to 7, wherein the content of water in dimethyl carbonate used in step (i) is controlled below 0.2% by weight.

9. A process according to any of claims 1 to 8, wherein a crude liquid of the urethane compound produced as the product of step (i) is neutralized with phosphoric acid and preferably is then washed with water.

10. A process according to claim 9, wherein the crude liquid neutralized with phosphoric acid is subjected to flash distillation.

11. A process according to any of claims 1 to 10, wherein the content of water in the aliphatic diamine used in step (i) is controlled below 1% by weight.

12. A process according to any of claims 1 to 11, wherein a crude liquid of the urethane compound produced as the product of step (i) is washed with a benzene/water system.

13. A process according to claim 12, wherein the crude liquid washed with a benzene/water system is fused and then subjected to purification by distillation.

14. A process according to any of claims 1 to 13, wherein methanol formed in step (ii) is removed through a vacuum line maintained under a pressure lower than 6,666 Pa (50 Torr).

15. A process compound according to any of claims 1 to 14, wherein the high-boiling-point solvent used in step (ii) is partially hydrogenated triphenyl.

16. A process according to any of claims 1 to 15, wherein the catalyst used in step (ii) is manganese acetate.

17. A process according to any of claims 1 to 15, wherein the catalyst used in step (ii) is selected from manganese sulfate, manganese chloride and manganese naphthenate.

18. A process according to any of claims 1 to 15, wherein the catalyst used in step (ii) is zinc bromide.

19. A process according to any of claims 1 to 18, wherein, in step (ii), the catalyst is continuously added in the form of a solution in methanol.

20. A process according to any one of claims 1 to 19, wherein the reaction of step (ii) is carried out in the presence of a triester of phosphorus acid.

21. A process according to claim 20, wherein the triester of phosphorous acid is triphenyl phosphite.

22. A process according to claim 20 or 21, wherein the amount of the triester of phosphorous acid is such that the molar ratio of the triester of phosphorous acid to the urethane compound is from 1/1,000 to 1/100.

## Patentansprüche

1. Ein Verfahren zur Herstellung einer aliphatischen Diisocyanat-Verbindung, enthaltend die Stufen:
(i) Umsetzen von Dimethylcarbonat mit einem aliphatischen Diamin in Gegenwart eines alkalischen Katalysators zur Herstellung der entsprechenden Urethan-Verbindung und dann
(ii) thermischer Abbau der Urethan-Verbindung bei einer Temperatur von 150 bis 300°C unter verringertem Druck von 133,3 bis 93.320 Pa (1 bis 700 Torr) in einem Lösemittel mit einem Siedepunkt der höher ist als derjenige der hergestellten Diisocyanat-Verbindung in Gegenwart eines Katalysators, der sich aus wenigstens einem Metall, ausgewählt aus Mangan, Molybdän, Wolfram, Zink und Beryllium oder einer Verbindung dieser Metalle zusammensetzt.

2. Ein Verfahren nach Anspruch 1, worin das Dimethylcarbonat durch Umsetzen von Kohlenmonoxid und Sauerstoff mit Methanol hergestellt wird.

3. Ein Verfahren nach Anspruch 1 oder 2, worin das aliphatische Diamin ein cyclisches Diamin ist.

4. Ein Verfahren nach einem der Ansprüche 1 bis 3, worin das aliphatische Diamin Isophoron-Diamin ist.

5. Ein Verfahren nach einem der Ansprüche 1 bis 4, worin der in Stufe (i) eingesetzte alkalische Katalysator Natriummethylat ist.

6. Ein Verfahren nach Anspruch 5, worin das als Katalysator eingesetzte Natriummethylat kontinuierlich oder periodisch hinzugegeben wird.

7. Ein Verfahren nach einem der Ansprüche 1 bis 6, worin das Molverhältnis eingesetztes Dimethylcarbonat/aliphatisches Diamin in Stufe (i) 2 bis 50 beträgt.

8. Ein Verfahren nach einem der Ansprüche 1 bis 7, worin der Wassergehalt des in Stufe (i) verwendeten Dimethylcarbonats unter 0,2 Gew.% gehalten wird.

9. Ein Verfahren nach einem der Ansprüche 1 bis 8, worin eine unbearbeitete Flüssigkeit der Urethan-Verbindung, die als Produkt der Stufe (i) hergestellt worden ist, mit Phosphorsäure neutralisiert und vorzugsweise dann mit Wasser gewaschen wird.

10. Ein Verfahren nach Anspruch 9, worin die unbearbeitete, mit Phoshorsäure neutralisierte Flüssigkeit einer Flash-Destillation unterzogen wird.

11. Ein Verfahren nach einem der Ansprüche 1 bis 10, worin der Wassergehalt des in Stufe (i) eingesetzten aliphatischen Diamins unter 1 Gew.% gehalten wird.

12. Ein Verfahren nach einem der Ansprüche 1 bis 11, worin eine unbearbeitete Flüssigkeit mit der als Produkt der Stufe (i) gebildeten Urethan-Verbindung mit einem System aus Benzol/Wasser gewaschen wird.

13. Ein Verfahren nach Anspruch 12, worin die unbearbeitete mit einem System aus Benzol/Wasser gewaschene Flüssigkeit vermischt und anschließend einer Reinigung durch Destillation unterzogen wird.

14. Ein Verfahren nach einem der Ansprüche 1 bis 13, worin das in Stufe (ii) gebildete Methanol im Vakuum unter einem Druck von weniger als 6,666 Pa (50 Torr) entfernt wird.

15. Ein Verfahren nach einem der Ansprüche 1 bis 14, worin das hochsiedende, in Stufe (ii) verwendete Lösemittel partialhydriertes Triphenyl ist.

16. Ein Verfahren nach einem der Ansprüche 1 bis 15, worin der in Stufe (ii) eingesetzte Katalysator Manganacetat ist.

17. Ein Verfahren nach einem der Ansprüche 1 bis 15, worin der in Stufe (ii) eingesetzte Katalysator ausgewählt ist aus Mangansulfat, Manganchlorid, Mangannaphtenat.

18. Ein Verfahren nach einem der Ansprüche 1 bis 15, worin der in Stufe (ii) verwendete Katalysator Zinkbromid ist.

19. Ein Verfahren nach einem der Ansprüche 1 bis 18, worin in Stufe (ii) der Katalysator kontinuierlich in Form einer methanolischen Lösung hinzugefügt wird.

20. Ein Verfahren nach einem der Ansprüche 1 bis 19, worin die Reaktion von Stufe (ii) in Gegenwart eines Phosphorsäuretriesters durchgeführt wird.

21. Ein Verfahren nach Anspruch 20, worin der Phosphorsäuretriester Triphenylphosphit ist.

22. Ein Verfahren nach Anspruch 20 oder 21, worin die Menge des Phosphorsäuretriesters derart ist, daß das Molverhältnis des Rhosphorsäuretriesters zur Urethan-Verbindung 1/1000 bis 1/100 beträgt.

## Revendications

1. Procédé de préparation d'un composé diisocyanate aliphatique, qui comprend les étapes consistant à :
(i) faire réagir du carbonate de diméthyle avec une diamine aliphatique en présence d'un catalyseur alcalin pour obtenir un composé uréthane correspondant, puis
(ii) décomposer thermiquement le composé uréthane à une température de 150 à 300°C, sous une pression réduite de 133,3 à 93320 Pa (1 à 700 torrs), dans un solvant présentant un point d'ébullition supérieur à celui du composé diisocyanate produit, en présence d'un catalyseur composé d'au moins un élément métallique choisi parmi le manganèse, le molybdène, le tungstène, le zinc et le béryllium ou d'un composé dudit métal.

2. Procédé selon la revendication 1, dans lequel on prépare le carbonate de diméthyle en faisant réagir du monoxyde de carbone et de l'oxygène avec du méthanol.

3. Procédé selon la revendication 1 ou la revendication 2, dans lequel la diamine aliphatique est une diamine cyclique.

4. Procédé selon l'une quelconque des revendications 1 à 3, dans lequel la diamine aliphatique est l'isophoronediamine.

5. Procédé selon l'une quelconque des revendications 1 à 4, dans lequel le catalyseur alcalin utilisé dans l'étape (i) est le méthylate de sodium.

6. Procédé selon la revendication 5, dans lequel on ajoute continuellement ou de manière intermittente le méthylate de sodium utilisé comme catalyseur.

7. Procédé selon l'une quelconque des revendications 1 à 6, dans lequel le rapport molaire carbonate de diméthyle/diamine aliphatique utilisé dans l'étape (i) est compris entre 2 et 50.

8. Procédé selon l'une quelconque des revendications 1 à 7, dans lequel on règle la teneur en eau du carbonate de diméthyle utilisé dans l'étape (i) au dessous de 0,2% en poids.

9. Procédé selon l'une quelconque des revendications 1 à 8, dans lequel on neutralise un liquide brut du composé uréthane produit dans l'étape (i) avec de l'acide phosphorique et de préférence, est ensuite lavé à l'eau.

10. Procédé selon la revendication 9, dans lequel on fait subir une distillation éclair au liquide brut neutralisé avec de l'acide phosphorique.

11. Procédé selon l'une quelconque des revendications 1 à 10, dans lequel on règle la teneur en eau de la diamine aliphatique utilisée dans l'étape (i) au dessous de 1% en poids.

12. Procédé selon l'une quelconque des revendications 1 à 11, dans lequel on lave un liquide brut du composé uréthane produit dans l'étape (i) avec un système benzène/eau.

13. Procédé selon la revendication 12, dans lequel on fond le liquide brut lavé avec un système benzène/eau puis on lui fait subir une purification par distillation.

14. Procédé selon l'une quelconque des revendications 1 à 13, dans lequel on enlève le méthanol formé dans l'étape (ii) par une canalisation de vide maintenue sous une pression inférieure à 6,666 Pa (50 torrs)

15. Procédé selon l'une quelconque des revendications 1 à 14, dans lequel le solvant de point d'ébullition élevé utilisé dans l'étape (ii) est du triphényle partiellement hydrogéné.

16. Procédé selon l'une quelconque des revendications 1 à 15, dans lequel le catalyseur utilisé dans l'étape (ii) est de l'acétate de manganèse.

17. Procédé selon l'une quelconque des revendications 1 à 15, dans lequel le catalyseur utilisé dans l'étape (ii) est choisi parmi le sulfate de manganèse, le chlorure de manganèse et le naphténate de manganèse.

18. Procédé selon l'une quelconque des revendications 1 à 15, dans lequel le catalyseur utilisé dans l'étape (ii) est du bromure de zinc.

19. Procédé selon l'une quelconque des revendications 1 à 18, dans lequel, dans l'étape (ii), on ajoute continuellement le catalyseur sous forme d'une solution dans le méthanol.

20. Procédé selon l'une quelconque des revendications 1 à 19, dans lequel on met en oeuvre la réaction de l'étape (ii) en présence d'un triester de l'acide phosphoreux.

21. Procédé selon la revendication 20, dans lequel le triester de l'acide phosphoreux est le phosphite de triphényle.

22. Procédé selon la revendication 20 ou la revendication 21, dans lequel la quantité de triester de l'acide phosphoreux est telle que le rapport molaire du triester de l'acide phosphoreux au composé uréthane soit de 1/1000 à 1/ 100.
